(19) Europäisches Patentamt / European Patent Office / Office européen des brevets

(11) **EP 1 753 393 B1**

(12) **FASCICULE DE BREVET EUROPEEN**

(45) Date de publication et mention de la délivrance du brevet:
**14.12.2016 Bulletin 2016/50**

(21) Numéro de dépôt: **04816488.3**

(22) Date de dépôt: **23.12.2004**

(51) Int Cl.:
*A61Q 1/14* $^{(2006.01)}$     *A61Q 19/08* $^{(2006.01)}$
*A61K 8/34* $^{(2006.01)}$     *A61K 8/60* $^{(2006.01)}$
*A61Q 19/00* $^{(2006.01)}$

(86) Numéro de dépôt international:
**PCT/FR2004/003376**

(87) Numéro de publication internationale:
**WO 2005/063194 (14.07.2005 Gazette 2005/28)**

(54) **PROCEDE D'INNOFORMULATION D'UNE BASE GALENIQUE BIOCOMPATIBLE**

VERFAHREN ZUR INNOFORMULIERUNG EINER BIOKOMPATIBLEN GALENISCHEN BASE

METHOD FOR THE INNOFORMULATION OF A BIOCOMPATIBLE GALENIC BASE

(84) Etats contractants désignés:
**AT BE BG CH CY CZ DE DK EE ES FI FR GB GR HU IE IS IT LI LT LU MC NL PL PT RO SE SI SK TR**

(30) Priorité: **23.12.2003 FR 0315290**

(43) Date de publication de la demande:
**21.02.2007 Bulletin 2007/08**

(73) Titulaire: **THOREL, Jean-Noël**
**75014 Paris (FR)**

(72) Inventeurs:
• **THOREL, Jean-Noel**
**75014 Paris (FR)**
• **REDZINIAK, Gérard**
**92160 Antony (FR)**

(74) Mandataire: **Cabinet Laurent & Charras**
**Le Contemporain**
**50 Chemin de la Bruyère**
**69574 Dardilly Cedex (FR)**

(56) Documents cités:
**EP-A- 0 943 324     FR-A- 2 838 055**
**US-A- 5 861 440**

• **PATENT ABSTRACTS OF JAPAN vol. 1998, no. 11, 30 septembre 1998 (1998-09-30) & JP 10 158151 A (ZERIA PHARMACEUT CO LTD), 16 juin 1998 (1998-06-16)**
• **PATENT ABSTRACTS OF JAPAN vol. 2002, no. 10, 10 octobre 2002 (2002-10-10) & JP 2002 161015 A (KOSE CORP), 4 juin 2002 (2002-06-04)**
• **PATENT ABSTRACTS OF JAPAN vol. 2000, no. 07, 29 septembre 2000 (2000-09-29) & JP 2000 103728 A (SHISEIDO CO LTD), 11 avril 2000 (2000-04-11)**

**Description**

La tolérance et l'innocuité des produits créés pour les êtres vivants, leur environnement et leur sécurité d'utilisation sont plus que jamais d'actualité, quel que soit le domaine. En matière de formulation cosmétique et encore plus dans le domaine des formulations dermo-cosmétiques, la prise en compte de ces facteurs est essentielle. Les produits dermo-cosmétiques sont souvent de par leur destination appliqués principalement sur des peaux fragilisées et réactives. L'obligation de neutralité est donc encore plus importante que pour les produits cosmétiques classiques, comme les produits de soin ou de maquillage.

En effet, les maladies de la peau ont en général pour conséquence biologique un affaiblissement de l'écosystème cutané et un accroissement de la sensibilité de la peau aux agents externes. Dans les problèmes de réactivité ou de dysfonctionnement liés à ces agents externes interviennent certains éléments contenus dans les bases cosmétiques, notamment les tensio-actifs, les conservateurs et la qualité de l'eau utilisée.

Dans une formulation cosmétique et/ou dermo-cosmétique destinée à développer une émulsion, une crème, un lait, voire une lotion ou une huile, on distingue classiquement deux éléments, à savoir, d'une part la base ou support, appelée galénique (objet de la présente invention), et d'autre part le ou les actifs cosmétiques, dermo-cosmétiques ou médicamenteux.

La base galénique dermique et/ou cosmétique n'a dans l'état de l'art aucune vocation biologique, elle constitue uniquement un véhicule ou support destiné :

- à véhiculer l'actif au bon endroit,
- à apporter à l'utilisateur ou consommateur les effets sensoriels qu'il attend par rapport à sa destination (yeux, corps, visage), sa forme (lait, crème), et à son parfum,
- à stabiliser et conserver le produit cosmétique et les actifs appropriés,
- à assurer la ou les fonctions de base attendues, en garantissant la plus grande compatibilité possible avec tout type de produits et de complexes actifs,

Et elle doit de plus, avoir une excellente tolérance vis-à-vis de la peau.

En cosmétique et/ou dermo-cosmétique, les bases sont principalement constituées de deux phases, une phase aqueuse et une phase grasse, dans lesquelles sont ajoutés des émulgateurs, et des agents techniques de stabilisation et de conservation, ainsi que des actifs cosmétiques ou pharmaceutiques.

Ces phase peuvent être uniques et continues, phase aqueuese continue, lotion, phase grasse continue, huile ou en mélange, lotion biphasique, mousse, émulsion multiples crèmes, mousses.

Cette base elle-même peut parfois, lorsqu'elle est appliquée sur une peau fragilisée et/ou réactive, provoquer par elle-même des réactions d'irritation et des phénomènes d'intolérance cutanée, qui vont s'ajouter aux réactions provoquées par les actifs, ou dus aux pathologies cutanées déjà présentes.

La base galénique utilisée comme véhicule se doit donc d'être parfaitement tolérée, et ce quels que soient l'état de la peau et les actifs vehiculés.

La présente invention permet de résoudre l'ensemble des problèmes ci-dessus évoqués, et concerne une base galénique dermique et/ou cosmétique à très haute tolérance, et parfaitement tolérée par la peau, quels que soient les ingrédients actifs et les additifs usuels utilisés en cosmétique et/ou dermo-cosmétique qui y sont incorporés et quels que soient les pathologies de la peau.

Cette base est définie pour respecter l'écosystème de la peau et être biocompatible avec les actifs cosmétiques et/ou médicamenteux, et avec l'état biologique de peaux fragilisées par la maladie.

Cette base galénique dermique et/ou cosmétique, en améliorant notamment la viabilité cellulaire, permet d'obtenir une résistance accrue aux ajouts externes et une hydratation, optimales, et donc une peau moins réactive. Elle permet également une diminution des phénomènes allergènes, tout en contenant des quantités réduites de conservateurs, qui peuvent également être une source de réactions d'intolérance.

On connaît de FR2609309 ou de EP1354580 ou de J20060893 l'utilisation en tant que principe actif cosmétique, de polyol ou oses comme par exemple comme substance stimulant cellulaire, comme source d'énergie comme antioxydant, mais les polyols n'ont jamais été décrits comme constituants d'une base galénique dermique et/ou cosmétique.

La présente invention concerne une base galénique dermique et/ou cosmétique, caractérisée en ce qu'elle contient dans sa phase aqueuse, au moins deux polyols choisis chacun dans le groupe constitué par les osides, oses ou produits de réduction des oses.

Elle concerne une base galénique dermique et/ou cosmétique, contenant dans sa phase aqueuse, au moins deux polyols choisis chacun dans le groupe constitué par les osides, oses ou produits de réduction des oses et caractérisée en ce que deux de ces polyols sont choisis dans le groupe des produits de réduction des oses constitué par le mannitol et le xylitol, et en ce qu'un polyol est choisi dans le groupe des oses constitué par le rhamnose, et en ce qu'un polyol est choisi dans le groupe des osides constitué par les fructooligosaccharides.

La phase aqueuse selon l'invention permet également d'améliorer la viabilité cellulaire de culture de Fibroblaste et Kératinocytes, par rapport à une phase aqueuse classique.

De plus la phase grasse peut en elle-même parfois avoir des effets négatifs sur la peau, notamment par action des lipoperoxydes qui pourraient s'y former. Ainsi pour garantir la haute tolérance maintenue par la phase aqueuse, lorsque la formulation comporte une phase grasse, une substance dite lipo-régulatrice est additionnée.

Cette susbstance lipo-régulatrice permet de rééquilibrer les éléments lipidiques de la peau et d'optimiser la tolérance sur des peaux déficientes.

Cette substance lipo-régulatrice en s'incorporant dans les structures moléculaires cutanées (membranes cellulaires, ciment intercellulaire épidermique) abaisse le seuil de réactivité de la peau.

L'invention concerne ainsi une base galénique dermique et/ou cosmétique selon l'invention caractérisée en ce qu'elle comporte en outre une phase grasse comportant une substance choisie parmi les substances lipo-régulatrices

Elle concerne plus particulièrement une base galénique dermique et/ou cosmétique selon l'invention, caractérisée en ce que la substance lipo-régulatrice est choisie parmi :

- les extraits lipidiques de plancton ou d'algues, comme Laminaria ochroleuca, riches en acide eicosapentaénoïque et en acide docosahexaénoïque.
- les huiles végétales contenant des triglycérides riches en acide alpha-linolénique, comme l'huile de colza, de soja, de lin,
- les huiles de poisson riches en acide alpha-linolénique, eicosapentaénoïque et docosahexaénoïque,
- les produits obtenus par chimie de synthèse ou de biosynthèse, de types mono, di ou triglycérides, ainsi que des phospho- et glyco-lipides dont la composition en acides gras se situe entre 10 et 100%, en acides alpha linolénique, eicosapentaénoïque et docosahexaénoïque.

Les éléments constitutifs de cette base galénique dermique et/ou cosmétique permettent de la rendre neutre vis-à-vis de la peau, et de garantir une parfaite innocuité vis-à-vis de celle-ci, c'est-à-dire lui permettent de respecter l'intégrité de celle-ci et de la rendre particulièrement adaptée aux peaux réactives. Ces résultats sont obtenus par une amélioration et/ou augmentation de la tolérance, à la fois de la phase aqueuse et de la phase grasse, par amélioration de la viabilité cellulaire, ainsi que par la non-réactivité des composants, c'est-à-dire leur neutralité vis-à-vis des actifs et des ingrédients classiquement utilisés en cosmétique et en dermo-cosmétique.

Les éléments constitutifs d'une base selon l'invention permettent également de maintenir et de rétablir l'homéostasie cutanée, y compris pour les peaux dans un état pathologique, et favorisent, lorsque des actifs sont incorporés, la réception de ces derniers par la peau.

Les bases galéniques dermiques et/ou cosmétiques de la présente invention conservent leur niveau de tolérance, quels que soient l'actif ou les actifs incorporés et véhiculés, et préservent des altérations cellulaires cutanées liées aux facteurs environnementaux.

Les bases galéniques dermiques et/ou cosmétiques selon l'invention, à savoir la phase aqueuse et/ou la phase grasse, sont par leur teneur respective en polyols ou en substances lipo-régulatrices utiles pour garantir l'écosystème général de la peau.

De préférence, une base galénique dermique et/ou cosmétique selon l'invention est caractérisée en ce que la teneur totale en polyols est comprise entre 0,1 et 40 % en poids total de la phase aqueuse.

Lorsque la base galénique comport une phase grasse selon l'invention, la teneur totale en substances liporégulatrices est comprise entre 0,01 et 100 % en poids total de la phase grasse.

On entend par polyol, un composé organique hydrocarboné possédant plusieurs groupes hydroxyles.

On entend par ose, un glucide contenant de 3 à 8 atomes de carbone, tous porteurs d'un groupe fonctionnel caractéristique oxygéné, groupe hydroxyle, cétone et/ou aldéhyde.

On entend par oside, un composé de la famille des glucides, produit de condensation avec élimination d'eau, de molécules d'oses ou de dérivés d'oses, liées entre elles par des liaisons glycosidiques.

On entend par produit de réduction des oses, des polyols linéaires obtenus par réduction de la fonction aldéhyde, qui cyclise un ose.

On entend par substance « lipo-régulatrice », une substance lipidique riche en acides gras poly-insaturés de type omega 3 et/ou oméga 6 (notamment acide alpha et gamma linolénique, acide eicosapentaenoique, acide docosahexaénoïque), qui en s'incorporant dans les structures moléculaires cutanées (membrane cellulaire, ciment intercellulaire épidermique) permettent d'abaisser significativement le seuil de réactivité de la peau.

La présente invention concerne également l'utilisation d'au moins deux polyols choisis chacun dans le groupe constitué par les osides, oses ou produits de réduction des oses et caractérisée en ce que deux de ces polyols sont choisis dans le groupe des produits de réduction des oses constitué par le mannitol et le xylitol, en ce qu'un polyol est choisi dans le groupe des oses constitué par le rhamnose, et en ce que le polyol est choisi dans le groupe des osides constitués par les fructooligosaccharides dans la phase aqueuse d'une base galénique dermique et/ou cosmétique, pour en améliorer la tolérance et optimiser les effets des actifs.

La présente invention est maintenant exposée au plan expérimental.

Mise en évidence de l'amélioration de la tolérance :

On a vérifié les proriétés d'amélioration de la tolérance par les polyols tels que précédemment définis, par un test permettant de mettre en évidence la non-altération de la fonction allostimulante des cellules de Langerhans épidermiques humaines.

Les polyols ont été mis en solution à une concentration de 2mg/ml dans un support.

Les supports testés, à savoir le xylitol, le rhamnose, le mannitol et le fructooligosaccharide ont été testés en culture mixte lympho-épidermique, seuls ou ensemble, aux concentrations finales de 1 et 10 %.

Le test a été conduit selon le protocole décrit dans « Human in vitro T cell sensitization using hapten-modified epidermal Langerhans cells » Advances in experimental medicine and biology, 1993, 209, pp 212, C. Moulon et al.

Des essais préliminaires de viabilité des cellules de Langerhans après 18 heures d'incubation en présence des différents produits n'ont pas montré d'effet toxique aux doses utilisées.

Les résultats des trois expériences réalisées avec des cellules provenant de différents donneurs montrent que les différents produits aux doses de 1 ou 10 % ne modifient pas de façon significative la fonction allostimulante de cellules de Langerhans. On observe seulement une légère diminution de cette fonction dans une expérience sur 3, lorsque les polysaccharides sont ajoutés ensemble à la culture mixte lympho-épidermique, à la dose de 10 %. L'interleukine 10 (IL10), connue pour son effet immunosuppresseur, est utilisée à titre de contrôle.

Les résultats montrent que dans des conditions normales, les supports des différents polyols testés ne modifient pas la fonction allostimulante des cellules de Langerhans épidermiques humaines.

Activité désensibilisante :

On observe l'activité désensibilisante de l'extrait lipidique de Laminaria ochroleuca, par abaissement du seuil de réactivité provoquée par une molécule irritante, à savoir le DNFB (di-nitro-fluoro-benzène).

Cette activité de l'extrait lipidique de Laminaria ochroleuca a été vérifiée par dissolution de l'extrait lipidique de Laminaria ochroleuca à 2 %, dans de la phase grasse d'une base galénique dermique et/ou cosmétique selon l'invention. La composition obtenue a été appliquée sur un expérimentateur à raison de 12,5 µl de crème matin et soir, pendant 3 jours.

Après 3 jours d'application, une dose irritante de DNFB (di-nitro-fluoro-benzène) (0,4 %) est appliquée, et l'oedème est mesuré à 3, 6, 9 et 24 heures après application. Les résultats obtenus montrent une diminution de l'oedème sur une peau, après application de la base avec l'extrait lipidique de Laminaria ochroleuca, en comparaison de l'application de la même dose de DNFB sans cette base..

Viabilité cellulaire :

La viabilité cellulaire des fibroblastes est appréciée par la technique de conversion du WST-1(*), qui consiste à évaluer l'activité du système mitochondrial succinate-tetrazolium reductase des cellules vivantes.

Le WST-1 (Boehringer/Roche) est réduit en un précipité coloré de formazan. La viabilité cellulaire est déterminée par lecture spectrophotométrique à 450nm. L'intensité de la densité optique est proportionnelle au nombre de cellules vivantes

(*) :Sel de tetrazolium WST-1 : (4-(3-(4-Iodophenyl)-2-(4-nitrophenyl)-2H-5-tetrazolio)-1,3-benzene disulfonate)

Ensemencement

Les fibroblastes sont ensemencés dans des microplaques de 96 puits à raison de 10 000 cellules par puits dans 200µl de milieu standard DMEM (SIGMA) enrichi en facteurs de croissance (10% SVF). Les plaques sont incubées 10 min puis 2 à 4 h, à 37°C, en atmosphère humide contenant 6% de CO2.

Gamme de concentrations testées

Les différentes concentrations à tester sont préparées à partir d'une solution -mère des polyols selon l'invention à 0,1 g/l jusqu'à 10g/l dans de l'eau.

Traitement

Après élimination du milieu DMEM, les différentes dilutions de produit sont mises au contact des cellules. Les milieux ne sont pas renouvelés au cours de l'expérience. Chaque point est réalisé en triplicate.

La cytotoxicité (test au WST-1) est mesurée après 10 min et 2 et 4 h de contact.

La densité optique est lue au lecteur de microplaque ELISA à 450nm.

|  | 0.1 g/l | 1g/l | 10 g/l | Eau distillée |
|---|---|---|---|---|
| Viabilité 10 min | 100 | 100 | 100 | 5 |
| 2h | 98 | 95 | 101 | 0 |
| 4h | 65 | 70 | 85 | 0 |

Les résultats montrent une augmentation de la viabilité pour des solutions contenant les polyols incorporés dans la phase aqueuse de la base galénique dermique et/ou cosmétique selon l'invention, et ce même après 4 heures.

Etude de la tolérance cutanée et du pouvoir sensibilisant:

L'étude est effectuée selon la méthode de Marzulli-Maibach sur 50 volontaires soumis à l'application de patches dans le dos sur un d=site homolatéral et sur un site controlatéral.

Deux formulations ont été testées, les formulations «R04FF17» et « R04FF18 ». La référence « R04FF17» representant une base galénique de l'art antérieur, et la référence «R04FF18» représentant cette même base galénique modifiée selon l'invention.

R04FF17 : base galénique de l'art antérieur

| Huile minérale | 12.0 % |
|---|---|
| PEG-8 Stéarate | 6.00 % |
| Glyceryl stéarate | 2.00 % |
| PEG-100 Stéarate Glyceryl stéarate | 2.00 % |
| Cetyle alcohol | 2.00 % |
| Stéaric acide | 2.00 % |
| Beurre de Karité | 1.00 % |
| Sorbitan sesquioleate | 0.50% |
| Phenoxyethanol | 0.30 % |
| Propyl Paraben | 0.15 % |
| Metyl Paraben | 0.15 % |
| Butyl Paraben | 0.10 % |
| Allantoine | 0.10 % |
| Triethanol amine | 0.34 % |
| Ethyl Paraben | 0.07 % |
| Eau | qsp |

R04FF18: base galénique de l'art antérieur modifiée selon l'invention.

| Mannitol | 1 |
|---|---|
| Rhamnose | 0.5 |
| Xylitol | 5 |
| Fructo oligo saccharide | 5 |

1- Détermination du pouvoir sensibilisant

Le produit est considéré comme sensibilisant si un sujet au moins présente tous les signes suivants, quel que soit le côté de survenue :

- érythème avec ou sans oedème,
- prurit
- vésicule

Aucune manifestation significative d'intolérance n' a été observée par l'investigateur pour les deux bases testées, dans les conditions de l'étude.

2- Détermination de l'indice d'irritation Z

Z est calculé sur les paramètres érythème (incluant l'oedème) et desquamation selon la formule suivante :

$$Z = \frac{\Sigma \text{ (scores érythème + desquamation)produit} - \Sigma \text{(scores érythème + desquamation)témoin}}{\text{Nombre de sujets x Nombre de lectures}}$$

Les scores erythème et desquamation étant calculé de la façon suivante :

$$\text{score érythème = cotation érythème x nombre d'occurrences}$$

$$\text{Score desquamation = cotation desquamation x nombre d'occurrences}$$

Z est calculé pour la zone homolatérale et pour la zone controlatérale. La valeur a plus élevée est prise en compte pour l'interprétation.
La grille d'interprétation de Z est la suivante :

| Classe | Valeur de Z | Potentiel irritant |
|--------|-------------|--------------------|
| 1 | $Z < 0,01$ | Pratiquement nul |
| 2 | $0,01 \le Z \le 0,04$ | Très faible |
| 3 | $0,05 \le Z \le 0,09$ | Faible |
| 4 | $Z < 0,10$ | Modéré |

Le produit R04FF17 présente un indice d'irritation Z = 0,05.
Le produit R04FF18 présente un indice Z = 0,00.
La modifiaction de la base galénique selon l'invention améliore son score de manière très intéressante.
Selon l'invention, la base galénique dermique et/ou cosmétique a un pH voisin de celui de la peau, compris entre 4 et 7, et se présente sous une forme adaptée pour une application dermique.
Elles se présentent notamment sous forme de solutions aqueuses, ou huileuses, voir alcooliques, de dispersions, de gels, d'émulsions obtenues par dispersion d'une phase grasse dans une phase aqueuse, ou inversement, de suspensions, d'émulsions. Ces préparations sont préparées selon des protocoles habituellement utilisés dans le domaine de la formulation cosmétique et/ou dermo-cosmétique.
Notamment, la phase grasse peut comprendre tout corps gras usuellement utilisé dans le domaine d'application envisagé. On peut notamment citer les corps gras siliconés tels que les huiles, les gommes et les cires de silicone, ainsi que les corps gras non siliconés, tels que les huiles et les cires d'origine végétale, minérale, animale et/ou synthétique. Les huiles peuvent éventuellement être volatiles ou non volatiles.
La présente invention concerne donc également une composition cosmétique et/ou dermo-cosmétique caractérisée en ce qu'elle comprend une base galénique dermique et/ou cosmétique selon l'invetion telle que précédemment définie.
Ces bases cométiques seront classiquement utilisées pour préparer des crèmes de protection, de traitement ou de soin, des laits corporels, des lotions, des gels pour le soin, le nettoyage de la peau, ainsi que des produits de maquillage (fond de teint, mascara, rouge à lèvres par exemple), et des produits de nettoyage et de soin des cheveux (shampooing, lotion, crème).
De façon connue dans le domaine cosmétique et/ou dermo-cosmétique, les bases galéniques selon l'invention peuvent contenir les actifs hydrophiles et/ou lipophiles nécessaires à l'activité recherchée, et les adjuvants habituels dans les domaines cosmétiques, dermo-cosmétiques ou dermatologiques. Ces adjuvants sont par exemple, et de façon non limitative, des gélifiants hydrophiles et/ou lipophiles, des conservateurs, les antioxydants, les solvants, les parfums, les charges et les matières colorantes. Les quantités de ces différents adjuvants sont celles utilisées classiquement dans les domaines considérés, et par exemple de 0,01 à 20 % en poids total de la composition.

Une base galénique dermique et/ou cosmétique selon l'invention est maintenant illustrée par les exemples de formulations donnés ci-après ; les compositions ci-après sont pondérales.

Exemple 1: Base galénique dermique et/ou cosmétique pour crème destinée à des peaux fragilisées

| A-Phase Grasse | |
|---|---|
| Arachidyl alcohol/behenyl alcohol/arachidylglucoside) | 1 % |
| Stéarate de glycérol | 5 % |
| Extrait lipidique de Laminaria ochroleuca | 1 % |
| Squalane | 15 % |
| Alcool cétylique 2% | 2 % |

| B-Phase aqueuse | |
|---|---|
| Eau | qsp.100 % |
| Glycérine | 2.0 % |
| Hexylene glycol | 3.0 % |
| Gomme de xanthane | 0.5% |
| Conservateurs | qs |
| Carbomer | 0.35% |

| C-Ingrédients ajoutés dans l'émulsion, à une température inférieure à 50°C. | |
|---|---|
| Mannitol | 0.75 % |
| Fructo-oligo saccaharide | 5.0 % |
| Rhamnose | 0.3% |
| Xylitol | 1.0% |
| Eau | 2% |
| Eau | 1.5% |
| NaOH | 0.35% |

Exemple 2: Base galénique dermique et/ou cosmétique pour crème destinée à des peaux normales

| A-Phase Grasse | |
|---|---|
| Ceteareth-2 | 3,5 % |
| Ceteareth-21 | 2 à 4 % |
| Huile de germe de blé | 3 % |
| Cyclomethicone | 7 % |
| Octyl Palmitate | 8% |
| Extrait lipidique de Laminaria ochroleuca | 0,01 % |

B-Phase aqueuse

| Eau | qsp.100 % |
|---|---|
| Glycérine | 7.0 % |
| Hexylene glycol | 3.0 % |
| Conservateurs | qs |

C-Ingrédients ajoutés dans l'émulsion, à une température inférieure à 50°C.

| PCANa | 0,5 % |
|---|---|
| Mannitol | 0.5 % |
| Fructo-oligo saccharide | 3.0 % |
| Rhamnose | 0.1 % |
| Xylitol | 2.0 % |
| Hyaluronate de sodium | 0,1 % |
| Eau | 5 % |
| | |
| Tocopherol | 0,05 % |
| Palmitate de vitamine A | 0,1 % |
| Phospholipides | 0,5 % |
| Céramides 3 | 0,1 % |
| Polyacrylamide & $C_{14-13}$ isoparaffine & laureth-7 | 2 à 3,5 % |

Exemple 3: Base galénique dermique et/ou cosmétique pour crème et lait destinés aux peaux exposées à la lumière du soleil

A-Phase Grasse

| Monostéreate de Glycerol | 2% |
|---|---|
| PEG-100 stearate | 3% |
| C12-C15 alkyl benzoate | 10% |
| Extrait lipidique de Laminaria ochroleuca | 5% |
| Dimethicone | 5% |
| Acétate de tocophérol | 1 % |
| Octyl-triazone (Uvinul T150) | 1.5 % |
| Butyl Methoxy Dibenzoyl methane (Eusolex 9020) | 2.0 % |
| Acool cetostéarylique | 1% |

B-Phase aqueuse

| Eau | qsp.100 % |
|---|---|
| Conservateurs | 0.6 % |
| Glycérine | 7 % |
| Hexylene glycol | 3.0 % |
| Carbomer | 0.5 % |
| Tetra sodium EDTA | 0,2 % |

C-Ingrédients ajoutés dans l'émulsion, à une température inférieure à 50°C.

| Sérine | 0,2 % |
|---|---|
| Mannitol | 0.5 % |
| Fructo-oligo saccharide | 3.0 % |

8

(suite)

| | |
|---|---|
| Rhamnose | 0.1 % |
| Xylitol | 2.0 % |
| Hyaluronate de sodium | 0,1 % |
| | |
| Eau | 5 % |
| NaOH | 0.5% |
| | |
| Parfum | qs. |

Exemple 4 : Base galénique dermique et/ou cosmétique pour lait ou crème destinés à des peaux fragilisées par un agent irritant

A-Phase Grasse

| | |
|---|---|
| Extrait lipidique de Laminaria ochroleuca | 5% |
| Squalane | 5% |
| Alcool cetylique | 2% |
| Dimethicone | 5% |
| Octyl palmitate | 5% |

B-Phase aqueuse

| | |
|---|---|
| Butylène glycol | 0,5-4 % |
| Eau | qsp.100 % |
| Glycérine | 2.0 % |
| Hexylene glycol | 3.0 % |
| Biosaccharide gum 2 | 1.0% |
| Gomme de xanthane | 0.5% |
| Conservateurs | qs |

C-Ingrédients ajoutés dans l'émulsion, à une température inférieure à 50°C.

| | |
|---|---|
| Acide glycyrrhétinique | 0,1 - 1 % |
| Mannitol | 0.5 % |
| Fructo-oligo saccharide | 3.0 % |
| Rhamnose | 0.1 % |
| Xylitol | 2.0% |
| Eau | 2.3 % |
| | |
| Acétate de tocophérol | 0,1 à 1 % |
| Pyridoxine | 0,01 à 0,05 % |
| Palmitate de vitamine A | 0,01 à 1 % |
| d-Panthénol | 0,1 à 1 % |
| | |
| Acide citrique | 0,1 à 0,5 % |
| Gluconate de zinc | 0,1 à 1 % |
| Citrate trisodique | 1 à 2,5 % |
| L-Fucose | 0,01 à 1 % |
| Eau | 5% |

Exemple 5 : Base galénique dermique et/ou cosmétique pour crème ou lait pour peaux ayant des déficiences liées au vielllissement cutané.

### A-Phase Grasse

| | |
|---|---|
| Extrait lipidique de Laminaria ochroleuca | 0,5 % |
| Stéarate de glycéryle | 1 à 5 % |
| Acide stéarique | 1 à 5 % |
| Isononyl Isononanoate | 1 à 15 % |

### B-Phase aqueuse

| | |
|---|---|
| Eau | qsp.100 % |
| Glycérine | 3.0 % |
| Gomme de xanthane | 0.5 % |
| Conservateurs | qs |

### C-Ingrédients ajoutés dans l'émulsion, à une température inférieure à 50°C.

| | |
|---|---|
| Mannitol | 0.5% |
| Fructo-oligo saccharide | 3.0 % |
| Rhamnose | 0.1% |
| Xylitol | 2.0% |
| Eau | 7.8 % |
| Pyridoxine | 0,01 à 0,05 % |
| Acide citrique | 0,1 à 0,5 % |
| Gluconate de zinc | 0,1 à 1% |
| Citrate trisodique | 1 à 2,5 % |
| Eau | 2% |
| d-Panthénol | 0,1 à 1 % |
| Palmitate de vitamine A | 0,01 à 1 % |
| Palmitate d'ascorbyle | 0,01 à 0,1 % |
| Acétate de tocophérol | 0,1 à 1 % |
| L-Fucose | 0,01 à 1 % |
| Lactoferrine / Lactoperoxydase | 0,01 à 1 % |
| Eau | 2 % |

Exemple 7 : Base galénique dermique et/ou cosmétique pour lotion isotonique

| | |
|---|---|
| Hexylene glycol | 4% |
| d-Panthénol | 0,1 % |
| Mannitol | 0.02 % |
| Fructo-oligo saccharide | 2.0% |
| Rhamnose | 0.01% |
| Xylitol | 0.50% |
| TriMethyl Glycine | 2 % |
| Conservateurs | qs |
| Eau | qsp.100 % |

Exemple 8 : Base galénique dermique et/ou cosmétique pour lotion démaquillante

| A-Phase aqueuse | |
|---|---|
| Polysorbate 20 | 1.0 % |
| Caprylyl/capryl glucoside (Oramix CG110) | 2.0 % |
| Extrait lipidique de Laminaria ochroleuca | 0,1 % |
| PEG-7 glyceryl cocoate | 0.5 % |
| Hexylene glycol | 4-5 % |
| d-Panthénol | 0,1 % |
| Mannitol | 0.02 % |
| Fructo-oligo saccharide | 1.0% |
| Rhamnose | 0.01 % |
| Xylitol | 0.50% |
| Conservateurs | qs |
| Eau | qsp.100 % |

## Revendications

1. Base galénique dermique et/ou cosmétique, contenant dans sa phase aqueuse, au moins deux polyols choisis chacun dans le groupe constitué par les osides, oses ou produits de réduction des oses et **caractérisée en ce que** deux de ces polyols sont choisis dans le groupe des produits de réduction des oses constitué par le mannitol et le xylitol, et **en ce qu'** un polyol est choisi dans le groupe des oses constitué par le rhamnose, et **en ce qu'**un polyol est choisi dans le groupe des osides constitué par les fructooligosaccharides.

2. Base galénique dermique et/ou cosmétique selon la revendication 1, caractérisée en qu'elle comporte en outre une phase grasse comportant une substance choisie parmi les substances lipo-régulatrices.

3. Base galénique dermique et/ou cosmétique selon la revendication 2, **caractérisée en ce que** la substance choisie parmi les substances lipo-régulatrices est un extrait lipidique de Laminaria ochroleuca riche en acide eicosapentaénoïque et en acide docosahexaénoïque.

4. Base galénique dermique et/ou cosmétique selon la revendication 2, **caractérisée en ce que** la substance choisie parmi les substances lipo-régulatrices est l'huile de soja.

5. Base galénique dermique et/ou cosmétique selon la revendication 2, **caractérisée en ce que** la substance choisie parmi les substances lipo-régulatrices est l'huile de lin.

6. Base galénique dermique et/ou cosmétique selon la revendication 2, **caractérisée en ce que** la substance choisie parmi les substances lipo-régulatrices est l'huile de colza.

7. Base galénique dermique et/ou cosmétique selon la revendication 2, **caractérisée en ce que** la substance choisie parmi les substances lipo-régulatrices est une huile de poisson riche en acides alpha linolénique, eicosapentaénoïque et docosahexaénoïque.

8. Base galénique dermique et/ou cosmétique selon la revendication 2, **caractérisée en ce que** la substance choisie parmi les substances lipo-régulatrices est un produit obtenu par chimie de synthèse ou de biosynthèse de types mono, di ou triglycérides, ou un phospho- ou glyco- lipide dont la composition en acides gras se situe entre 10 et 100% en acide alpha linolénique, eicosapentaénoïque et docosahexaénoïque.

9. Utilisation d'au moins deux polyols choisis chacun dans le groupe constitué par les osides, oses ou produits de réduction des oses et **caractérisée en ce que** deux de ces polyols sont choisis dans le groupe des produits de réduction des oses constitué par le mannitol et le xylitol, **en ce qu'**un polyol est choisi dans le groupe des oses constitué par le rhamnose, et **en ce que** le polyol est choisi dans le groupe des osides constitués par les fructooligosaccharides dans la phase aqueuse d'une base galénique dermique et/ou cosmétique, pour en améliorer la tolérance.

10. Base galénique dermique et/ou cosmétique selon l'une quelconque des revendications 1 à 8, **caractérisée en ce que** la teneur totale en polyol est comprise entre 0,1 et 40 % en poids total de la phase aqueuse.

11. Base galénique dermique et/ou cosmétique selon la revendication 2, **caractérisée en ce que** la teneur totale en substances lipo-régulatrices est comprise entre 0,01 et 100 % en poids total de la phase grasse.

12. Composition cosmétique et ou dermo-cosmétique, **caractérisée en ce qu'**elle comprend une base selon l'une quelconque des revendications 1 à 8 et 10 à 11.

**Patentansprüche**

1. °/ Dermatologische und / oder kosmetische galenische Base, die in ihrer wässrigen Phase mindestens zwei Polyole enthält, ausgewählt jeweils aus der Gruppe bestehend aus Osiden, Osen oder Osen-Reduktionsprodukten und **dadurch gekennzeichnet, dass** zwei dieser Polyole ausgewählt werden aus der Gruppe der Osen-Reduktionsprodukte, bestehend aus Mannitol und Xylitol und dadurch, dass ein Polyol ausgewählt wird, aus der Gruppe der Osen bestehend aus Rhamnose und dadurch dass Polyol ausgewählt wird, aus der Gruppe der Oside, bestehend aus den Fructooligosacchariden.

2. °/ Dermatologische und / oder kosmetische galenische Base gemäß Anspruch 1, **dadurch gekennzeichnet, dass** sie außerdem eine Fettphase enthält, die eine Substanz enthält, die aus den fettregulierenden Substanzen ausgewählt wird.

3. °/ Dermatologische und / oder kosmetische galenische Base gemäß Anspruch 2, **dadurch gekennzeichnet, dass** die aus den fettregulierenden Substanzen ausgewählte Substanz ein an Eicosapentaensäure und Docosahexaensäure, reicher Lipidextrakt von Laminaria ochroleuca ist.

4. °/ Dermatologische und / oder kosmetische galenische Base gemäß Anspruch 2, **dadurch gekennzeichnet, dass** die aus den fettregulierenden Substanzen ausgewählte Substanz, Sojaöl ist.

5. °/ Dermatologische und / oder kosmetische galenische Base gemäß Anspruch 2, **dadurch gekennzeichnet, dass** die aus den fettregulierenden Substanzen ausgewählte Substanz, Leinöl ist.

6. °/ Dermatologische und / oder kosmetische galenische Base gemäß Anspruch 2, **dadurch gekennzeichnet, dass** die aus den fettregulierenden Substanzen ausgewählte Substanz, Rapsöl ist.

7. °/ Dermatologische und / oder kosmetische galenische Base gemäß Anspruch 2, **dadurch gekennzeichnet, dass** die aus den fettregulierenden Substanzen ausgewählte Substanz, ein an Alpha-Linolensäure, Eicosapentaensäure und Docosahexaensäure reiches Fischöl ist.

8. °/ Dermatologische und / oder kosmetische galenische Base gemäß Anspruch 2, **dadurch gekennzeichnet, dass** die aus den fettregulierenden Substanzen ausgewählte Substanz ein durch chemische Synthese oder Biosynthese erhaltenes Produkt ist, vom Typ Mono-, Di-, oder Tri-Glyzerid, oder ein Phospho- oder Glykolipid, dessen Fettsäurezusammensetzung zwischen 10 und 100 % an Alpha-Linolensäure, Eicosapentaensäure und Docosahexaensäure liegt.

9. °/ Verwendung von mindestens zwei Polyolen, ausgewählt jeweils aus der Gruppe bestehend aus Osiden, Osen oder Osen-Reduktionsprodukten und **dadurch gekennzeichnet, dass** zwei dieser Polyole ausgewählt werden aus der Gruppe der Reduktionsprodukte der Osen, bestehend aus Mannitol und Xylitol und dadurch, dass ein Polyol ausgewählt wird aus der Gruppe der Osen, bestehend aus Rhamnose und dadurch dass Polyol ausgewählt wird aus der Gruppe der Oside, bestehend aus Fructooligosacchariden in der wässrigen Phase einer dermatologischen und/oder kosmetischen galenischen Base, um die Toleranz zu verbessern.

10. °/ Dermatologische und / oder kosmetische galenische Base gemäß irgendeinem der Ansprüche 1 bis 8, **dadurch gekennzeichnet, dass** der Gesamtgehalt an Polyol zwischen 0,1 und 40 % des Gesamtgewichtes der wässrigen Phase liegt.

11. °/ Dermatologische und / oder kosmetische galenische Base gemäß Anspruch 2, **dadurch gekennzeichnet, dass** der Gesamtgehalt an fettregulierenden Substanzen zwischen 0,01 und 100 % des Gesamtgewichtes der Fettphase liegt.

12. °/ Kosmetische oder dermo-kosmetische Zusammensetzung, **dadurch gekennzeichnet, dass** sie eine Base gemäß irgendeiner der Ansprüche 1 bis 8 und 10 bis 11 enthält.

**Claims**

1. A dermal and/or cosmetic galenic base whose aqueous phase contains at least two polyols each selected from the group consisting of osides, oses or ose reduction products, and **characterized in that** two of these polyols are selected from the group of ose reduction products consisting of mannitol and xylitol, and **in that** a polyol is selected from the group of oses consisting of rhamnose, and **in that** a polyol is selected from the group of osides consisting of fructooligosaccharides.

2. The dermal and/or cosmetic galenic base according to claim 1, **characterized in that** it further contains a fatty phase comprising a substance selected from liporegulatory substances.

3. The dermal and/or cosmetic galenic base according to claim 2, **characterized in that** the substance selected from liporegulatory substances is a lipid extract of Laminaria ochroleuca which is rich in eicosapentaenoic acid and docosahexaenoic acid.

4. The dermal and/or cosmetic galenic base according to claim 2, **characterized in that** the substance selected from liporegulatory substances is soy oil.

5. The dermal and/or cosmetic galenic base according to claim 2, **characterized in that** the substance selected from liporegulatory substances is linseed oil.

6. The dermal and/or cosmetic galenic base according to claim 2, **characterized in that** the substance selected from liporegulatory substances is rapeseed oil.

7. The dermal and/or cosmetic galenic base according to claim 2, **characterized in that** the substance selected from liporegulatory substances in a fish oil rich in alpha-linolenic, eicosapentaenoic and docosahexaenoic acids.

8. The dermal and/or cosmetic galenic base according to claim 2, **characterized in that** the substance selected from liporegulatory substances is a product obtained by synthetic or biosynthetic chemistry of the mono-, di- or triglyceride type, or a phospholipid or glycolipid whose fatty acid composition is between 10 and 100% of alpha-linolenic, eicosapentaenoic and docosahexaenoic acids.

9. The use of at least two polyols each selected from the group consisting of osides, oses and ose reduction products, and **characterized in that** two of these polyols are selected from the group of ose reduction products consisting of mannitol and xylitol, and **in that** a polyol is selected from the group of oses consisting of rhamnose, and **in that** a polyol is selected from the group of osides consisting of fructooligosaccharides in the aqueous phase of a dermal and/or cosmetic galenic base, for improving its tolerability.

10. The dermal and/or cosmetic galenic base according to any of claims 1 to 8, **characterized in that** the total polyol content is between 0.1 and 40% of the total weight of the aqueous phase.

11. The dermal and/or cosmetic galenic base according to claim 2, **characterized in that** the total content of liporegulatory substances is between 0.01 and 100% of the total weight of the fatty phase.

12. A cosmetic and/or dermo-cosmetic composition, **characterized in that** it comprises a base according to any of claims 1 to 8 and 10 to 11.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2609309
- EP 1354580 A
- WO J20060893 A

**Littérature non-brevet citée dans la description**

- **C. MOULON.** Human in vitro T cell sensitization using hapten-modified epidermal Langerhans cells. *Advances in experimental medicine and biology,* 1993, vol. 209, 212